(19) Europäisches Patentamt
European Patent Office
Office européen des brevets

(11) **EP 3 415 118 B1**

(12) **FASCICULE DE BREVET EUROPÉEN**

(45) Date de publication et mention
de la délivrance du brevet:
**01.04.2020 Bulletin 2020/14**

(21) Numéro de dépôt: **18177192.4**

(22) Date de dépôt: **12.06.2018**

(51) Int Cl.:
***A61F 2/16*** *(2006.01)*

(54) **IMPLANT OPTIQUE INTRAOCULAIRE COMPORTANT UNE SURFACE DE TRANSITION**

INTRAOKULARES OPTISCHES IMPLANTAT, DAS EINE ÜBERGANGSOBERFLÄCHE UMFASST

INTRAOCULAR LENS IMPLANT INCLUDING A TRANSITION SURFACE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.06.2017 FR 1755515**

(43) Date de publication de la demande:
**19.12.2018 Bulletin 2018/51**

(73) Titulaire: **Cutting Edge
34000 Montpellier (FR)**

(72) Inventeur: **ORTUNO, Angel
31100 Toulouse (FR)**

(74) Mandataire: **Cabinet Beau de Loménie
51 avenue Jean Jaurès
BP 7073
69301 Lyon Cedex 07 (FR)**

(56) Documents cités:
**EP-A1- 2 979 663        EP-A1- 3 042 633
US-A1- 2007 106 381**

EP 3 415 118 B1

**Description**

L'invention concerne le domaine des implants optiques intraoculaires.

Les implants intraoculaires sont des lentilles optiques qui sont destinées à être implantées dans l'œil d'un patient pour corriger des anomalies optiques de l'œil. Ils peuvent être ainsi utilisés pour remédier à diverses anomalies telles que la myopie, l'hypermétropie, la presbytie, l'astigmatisme ou la cataracte.

Des tels implants peuvent être destinés à être implantés dans la chambre antérieure de l'œil, dans la chambre postérieure, voire, notamment dans le cas de la cataracte, à la place du cristallin.

Certains implants sont des implants unifocaux ou monofocaux qui focalisent la lumière venant de l'extérieur en un seul point focal. Ces implants unifocaux ont un niveau de performance élevé. Néanmoins ces implants unifocaux présentent une limitation importante qui réside dans le fait qu'ils ne permettent pas de recréer le phénomène d'accommodation du cristallin naturel. Ce phénomène d'accommodation permet de faire une mise au point et de rendre possible la vision de loin comme de près.

Plus récemment des évolutions d'implants monofocaux sont proposées afin d'offrir aux patients une vision plus étendue allant par exemple de la vision de loin à la vision intermédiaire. Ils sont généralement nommés implants avec profondeur de champ étendue. Ces implants ont une plage de focalisation, c'est-à-dire que les rayons lumineux traversant l'optique vont focaliser sur une zone plus au moins étendue en fonction de la conception de l'optique. Ils n'auront donc pas un point focal idéal parfaitement défini. De tels implants peuvent par exemple utiliser une surface optique induisant des aberrations sphériques.

Enfin, des implants multifocaux sont proposés qui focalisent la lumière venant de l'extérieur en plusieurs points focaux, généralement deux, pour corriger la vision de près et la vision de loin, ou trois pour corriger en plus la vision intermédiaire.

De manière générale, les implants multifocaux peuvent être divisés en deux grandes catégories : les implants multifocaux diffractifs et les implants multifocaux réfractifs.

Dans les implants multifocaux diffractifs, l'implant comporte des zones qui présentent une structuration de surface créant un réseau de diffraction. Ce réseau de diffraction peut comporter des facettes de diffraction dont au moins une dimension selon au moins une direction est inférieure à 1 millimètre.

Dans les implants multifocaux réfractifs, la surface active de l'implant est répartie en plusieurs zones macroscopiques, chaque zone macroscopique présentant deux dimensions maximum macroscopiques, selon deux directions perpendiculaires, qui sont chacune supérieures par exemple à 1 millimètre. Chaque zone macroscopique forme un dioptre optique réfractif au travers duquel les rayons lumineux sont déviés selon la loi de Snell-Descarte. Dans le cas d'un dioptre sphérique ou modérément asphérique, la totalité des rayons lumineux provenant de l'infini se focalisent sur un point focal F à une distance f d'un centre optique de l'implant, ce point étant associé à une zone macroscopique formant un dioptre fournissant une correction optique définie correspondant à ce point focal. Bien entendu, on peut avoir des implants multifocaux et à profondeur de champ étendue, dans lesquels on introduit, pour au moins une des zones macroscopiques, des aberrations sphériques.

Dans de nombreux cas, les implants multifocaux ou à profondeur de champs étendue, réfractifs, présentent différentes zones coaxiales, formant chacune un dioptre, les différentes zones fournissant ainsi chacune un jeu particulier de propriété optiques. Un tel implant à dioptres réfractifs coaxiaux comporte ainsi un dioptre central circulaire, comprenant le centre optique de l'implant, et au moins un dioptre périphérique, autour du dioptre central, de forme annulaire. Chaque dioptre présente un jeu particulier de propriétés optiques, les deux jeux étant distincts par au moins une propriété optique.

Toutefois, les implants à dioptres réfractifs coaxiaux connus jusqu'à présent des inconvénients au niveau des zones de raccordement entre les différents dioptres. En effet, ces zones de raccordement, sensiblement circulaires ou annulaires autour de l'axe principal correspondent à des ruptures de pente des surfaces annulaires de part et d'autre de la zone de raccordement. Ces ruptures de pentes se traduisent par une trajectoire non maîtrisée des rayons lumineux qui impactent l'implant au niveau de ces zones de raccordement. Cela peut se traduire par une gêne visuelle pour le porteur de l'implant. Un implant selon le préambule de la revendication 1 est connu du document EP-A-2 979 663.

L'invention a donc pour but de proposer une nouvelle conception d'un implant à dioptres réfractif coaxiaux qui permette une meilleure maîtrise de la trajectoire des rayons lumineux qui traversent ces zones de raccordement.

Dans ce but, l'invention propose un implant optique intraoculaire :

- du type comportant un corps optique réfractif présentant deux faces, respectivement arrière et avant, comportant chacune respectivement une surface optique primaire, respectivement arrière et avant, qui s'étendent chacune respectivement sur une surface de base primaire courbe, respectivement arrière et avant, les surfaces de bases primaires courbes définissant, par réfraction, un jeu primaire de propriétés optiques ;
- du type dans lequel, parmi les faces arrière ou avant, au moins une première face, comporte une première surface optique primaire s'étendant sur une première surface de base primaire courbe, et au moins une surface optique secondaire qui s'étend sur une surface de base secondaire courbe définissant, par réfraction, une jeu secondaire de propriétés optiques;

- du type dans lequel la première surface de base primaire est une surface de révolution autour d'un axe primaire ;
- et du type dans lequel la surface de base secondaire est une surface de révolution.

Un tel implant est caractérisé en ce que :

- la première face comporte, au moins sur un secteur angulaire de transition autour de l'axe primaire, une surface de transition qui s'étend, selon une direction radiale par rapport à l'axe primaire, entre la première surface optique primaire et la surface optique secondaire ;
- en ce qu'un plan de coupe considéré, contenant l'axe primaire, coupe respectivement la première surface optique primaire, la surface de transition et la surface optique secondaire respectivement selon un tronçon primaire, un tronçon de transition et un tronçon secondaire, les tronçons se raccordant dans cet ordre respectivement en un point de transition primaire et un point de transition secondaire ;

- en ce que dans le plan de coupe considéré, le tronçon primaire présente, au point de transition primaire, une tangente primaire ayant une orientation primaire, et au point de transition secondaire, le tronçon secondaire présente une tangente secondaire ayant une orientation secondaire ;
- et en ce que, dans tout plan de coupe considéré contenant l'axe primaire et compris dans le secteur angulaire de transition autour de l'axe primaire, la surface de transition présente, en tout point donné du tronçon de transition, une tangente locale de transition au tronçon de transition dont l'orientation est comprise dans la plage angulaire allant de l'orientation primaire à l'orientation secondaire.

Selon d'autres caractéristiques optionnelles d'un implant selon l'invention, prises seules ou en combinaison :

- Dans tout plan de coupe considéré contenant l'axe primaire et compris dans un secteur dudit secteur angulaire de transition autour de l'axe primaire, pour tout point donné du tronçon de transition, l'orientation de la tangente locale de transition évolue, le long du tronçon de transition depuis le point de transition primaire jusqu'au point de transition secondaire, selon une fonction continue de l'écartement radial du point donné, l'écartement radial étant la distance, selon une direction perpendiculaire à l'axe primaire, entre le point donné et l'axe primaire.
- Dans tout plan de coupe considéré contenant l'axe primaire et compris dans un secteur dudit secteur angulaire de transition autour de l'axe primaire, l'orientation de la tangente locale de transition au point de transition primaire est égale à l'orientation primaire.
- Dans tout plan de coupe considéré contenant l'axe primaire et compris dans un secteur dudit secteur angulaire de transition autour de l'axe primaire, l'orientation de la tangente locale de transition au point de transition secondaire est égale à l'orientation secondaire.
- Dans tout plan de coupe considéré contenant l'axe primaire et compris dans un secteur dudit secteur angulaire de transition autour de l'axe primaire, la surface de transition présente, en tout point donné du tronçon de transition depuis le point de transition primaire jusqu'au point de transition secondaire, une dérivée de la pente de la tangente locale de transition qui varie selon une fonction continue, de préférence polynomiale, de l'écartement radial dans un repère orthogonal et normé du plan de coupe considéré.
- Dans tout plan de coupe considéré contenant l'axe primaire et compris dans un secteur dudit secteur angulaire de transition autour de l'axe primaire, la surface de transition présente, en tout point donné du tronçon de transition depuis le point de transition primaire jusqu'au point de transition secondaire, une pente de la tangente locale de transition qui varie selon une fonction linéaire de l'écartement radial dans un repère orthogonal et normé du plan de coupe considéré.
- Dans tout plan de coupe considéré contenant l'axe primaire et compris dans un secteur dudit secteur angulaire de transition autour de l'axe primaire, le tronçon de transition est un tronçon de courbe conique.
- Dans le secteur angulaire de transition, la surface de transition s'étend sur une surface de révolution ayant comme axe de révolution l'axe primaire.
- Le secteur angulaire de transition s'étend sur 360 degrés d'angle autour de l'axe primaire.
- L'une parmi la surface optique primaire et la surface optique secondaire s'étend radialement vers l'extérieur autour de l'autre par rapport à l'axe primaire (01).
- L'une ou l'autre, ou les deux, parmi la surface optique primaire et la surface optique secondaire, est une surface de révolution autour de l'axe primaire.
- La surface de transition est de préférence comprise entre un cercle de transition primaire et un cercle de transition secondaire qui présentent une différence de rayon de 0.5 millimètre ou moins, de préférence 0.15 millimètre ou moins, plus préférentiellement comprise dans la gamme allant de 0.05 à 0.15 millimètres.
- La surface de transition est délimitée par et comprise entre un cercle de transition primaire commun avec la surface optique primaire et un cercle de transition secondaire commun avec la surface optique secondaire.

- Le cercle de transition primaire et le cercle de transition secondaire présentent une différence de rayon de 0.5 millimètre ou moins, de préférence 0.15 millimètre ou moins, plus préférentiellement comprise dans la gamme allant de 0.05 à 0.15 millimètres.
- La surface de base secondaire est une surface de révolution autour du même axe primaire que la première surface de base primaire.
- La première surface optique primaire et la surface optique secondaire sont toutes les deux des surfaces convexes, tandis que la surface de transition est une surface concave.

Diverses autres caractéristiques ressortent de la description faite ci-dessous en référence aux dessins annexés qui montrent, à titre d'exemples non limitatifs, des formes de réalisation de l'objet de l'invention.

La **Figure 1** est une vue schématique en plan d'un implant intraoculaire réfractif selon l'art antérieur.
La **Figure 2** est une vue schématique en coupe de l'implant de la **Fig. 1.**
La **Figure 3** est un agrandissement d'une partie de la **Fig. 2** correspondant à un raccordement entre surfaces optiques.
La **Figure 4** est une vue schématique en plan, partielle, similaire à celle de la **Fig. 1,** illustrant un premier mode de réalisation d'un implant intraoculaire réfractif selon l'invention.
La **Figure 5** est une vue schématique en coupe, selon un plan de coupe considéré, de l'implant selon l'invention illustré à la **Fig. 4.**
La **Figure 6** est un agrandissement d'une partie de la **Fig. 5** correspondant à une transition entre une surface optique primaire et une surface optique secondaire ;
La **Figure 7** est un schéma explicatif correspondant à la **Fig. 6.**
La **Figure 8** est une vue schématique en coupe d'un autre implant selon l'invention.

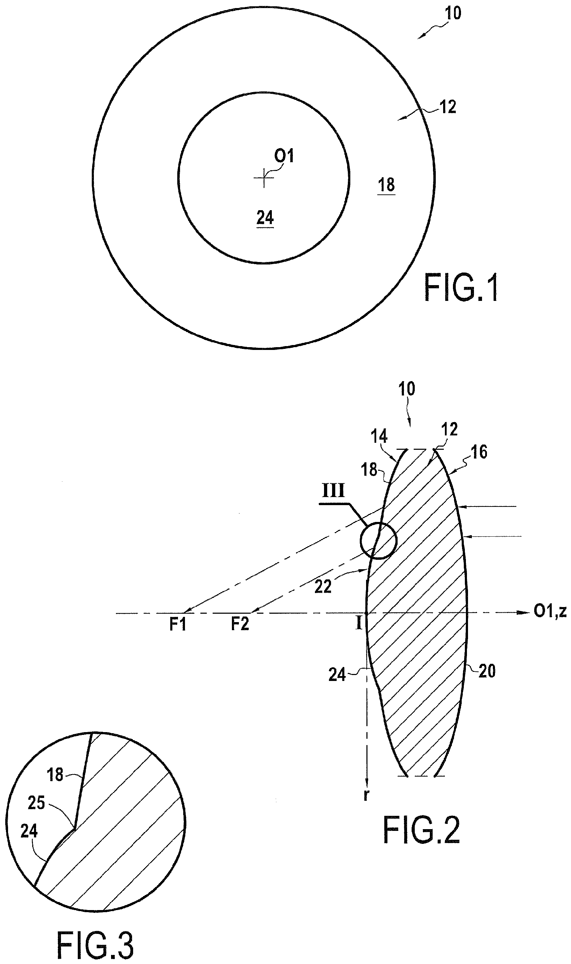

On a illustré sur les **Figs. 1** à **6,** de manière partielle, un implant intraoculaire selon l'art antérieur et un implant intraoculaire réfractif **10** conforme aux enseignements de l'invention. On a illustré uniquement la partie optique de l'implant, qui est généralement la partie centrale de l'implant utile à la vision, qui est la partie traversée par la lumière et qui influe sur la direction des rayons lumineux destinés à impacter la rétine. Cette partie centrale utile à la vision présente généralement un diamètre compris entre 5 et 8 millimètres, typiquement, un diamètre de 6 ou 7 millimètres. De manière connue, un implant comporte généralement des éléments de fixation et de maintien, aussi appelés partie haptique de l'implant, généralement disposés autour de la partie centrale optique, et qui peuvent prendre la forme d'éléments de plaques ou de pattes.

La partie centrale optique de l'implant comporte un corps optique **12** réfractif présentant deux faces, respectivement arrière **14** et avant **16.** De manière générale, les faces arrière **14** et avant **16** sont des faces de géométrie globale convexe. Cependant, on peut envisager que l'une ou l'autre de la face arrière et/ou de la face avant présente une géométrie globale concave ou plane. Dans la description qui va suivre, on considère que la face avant **16** de l'implant est celle qui est tournée vers la cornée de l'œil tandis que la face arrière **14** est celle qui est tournée vers la rétine. De même, les exemples de réalisation vont être décrits dans le cas où au moins un dispositif réfractif auxiliaire est disposé sur la face arrière **14** de l'implant. Toutefois, on peut prévoir au moins un dispositif réfractif auxiliaire sur la face avant **16** de l'implant, voire prévoir tous les dispositifs réfractifs auxiliaires sur la face avant **16** de l'implant. De même, on décrira des modes de réalisation dans lesquels tous les dispositifs réfractifs auxiliaires sont disposés sur une même face, en l'occurrence la face arrière **14,** mais on peut aussi prévoir que, pour un même implant **10,** un ou plusieurs dispositif(s) réfractif(s) auxiliaire(s) soi(en)t disposé(s) sur la face arrière **14** et un ou plusieurs dispositif(s) auxiliaire(s) soi(en)t disposé(s) sur la face avant **16** de l'implant.

De manière connue, le corps optique réfractif **12** est transparent à la lumière visible. Dans l'exemple illustré, on considère que le corps optique réfractif est constitué d'un unique matériau transparent. Le matériau utilisé pour la réalisation du corps optique réfractif est un matériau habituellement utilisé pour les implants optique intraoculaires. Il peut d'agir de matériaux rigides, ne permettant pas la déformation de l'implant pour l'implantation, ou, de préférence, de matériaux souples permettant la déformation de l'implant pour l'implantation. Parmi les matériaux souples, on peut citer :

- les élastomères silicones ;
- les polymères acryliques, tels que les copolymères synthétisés à partir de différents composants de la famille des acrylates ou méthacrylates. De tels polymères sont dits « hydrophiles » s'ils contiennent, en masse, plus de 15% d'eau, ou « hydrophobes » s'ils contiennent, en masse, moins de 5% d'eau, préférentiellement moins de 1% d'eau ;
- les polymères polyuréthanes.

De préférence, ce corps optique réfractif **12** est monobloc.

La face arrière **14** et la face avant **16** comportent chacune respectivement une surface optique primaire, respectivement arrière **18** et avant **20,** qui s'étendent chacune respectivement sur une surface de base primaire courbe, respectivement arrière et avant. La géométrie des surfaces de base primaires courbes **18, 20** définissent, en réfraction, un jeu primaire de propriétés optiques.

Dans la description qui suit, on considère que l'une des deux faces arrière **14** et avant **16** comporte une surface optique unique, s'étendant sur une surface de base courbe unique et continue, par exemple la face avant **16**. Dans ce cas, la surface optique primaire avant **20** est confondue avec la surface de base courbe primaire sur toute la superficie de la face considérée dans l'implant.

Toutefois, parmi les faces arrière **14** ou avant **16**, au moins une première face, comportant une première surface optique primaire s'étendant sur une première surface de base primaire courbe, est munie d'au moins un dispositif réfractif auxiliaire **22**, formé par une excroissance ou une dépression dans la première face par rapport à la première surface primaire de base de la première surface optique primaire. Par exemple, la première face est la face arrière **14**. La surface optique primaire arrière **18** est la surface optique qui s'étend sur une surface de base primaire arrière courbe.

Dans la suite du texte, une surface de base est une surface théorique qui correspond à la surface d'un corps optique ayant un unique jeu de propriétés optiques sur toute l'étendue optiquement utile du corps optique. Cette surface de base est définie selon la manière habituelle pour l'homme du métier souhaitant obtenir un jeu souhaité de propriétés optiques, par exemple une correction optique souhaitée, notamment une vergence souhaitée. La surface optique associée est une surface réelle du corps optique **12**, délimitée par un contour, et est une portion de la surface de base.

La première surface de base primaire est une surface de révolution autour d'un axe primaire **O1**.

De préférence, les surfaces optiques primaires **18**, **20**, sont aussi des surfaces de révolution dont l'axe de symétrie de révolution est l'axe primaire **O1**. Dans ces cas, elles présentent un contour circulaire

Ces surfaces optiques primaires, et donc les surfaces de base sur lesquels elles s'étendent, peuvent être des surfaces sphériques mais sont de préférence, de manière connue, des surfaces asphériques. Notamment, ces surfaces optiques primaires asphériques peuvent être des surfaces définies par leur flèche z en fonction de la distance r, ou écartement radial, à l'axe primaire **O1**, l'écartement radial étant la distance, selon une direction perpendiculaire à l'axe primaire **O1**, entre un point donné de la surface optique considérée et l'axe primaire **O1**.

De préférence, on définit le repère orthogonal et normé (**I, Iz, Ir**) où **I** est l'origine du repère, l'axe **Iz** correspond à l'axe primaire **O1**, sur lequel est mesurée la flèche z, et **Ir** est un axe perpendiculaire à l'axe **Iz** sur lequel est mesuré l'écartement radial r. Dans un tel repère of peut définir une surface asphérique comme l'ensemble des points respectant une fonction z(r) particulière.

De manière générale, cette fonction dépend pour l'essentiel de deux paramètres principaux :

- le rayon de courbure R (ou la courbure C = 1/R) ;
- et la conicité K,
- éventuellement additionnés d'une suite de termes polynomiaux d'ordre pair

Cette fonction peut s'écrire :

$$z(r) = \frac{r^2}{R\left(1 + \sqrt{1 - (1+\kappa)\frac{r^2}{R^2}}\right)} + \alpha_1 r^2 + \alpha_2 r^4 + \alpha_3 r^6 + \cdots$$

Suivant la valeur de conicité K, le profil prendra différentes formes :

| K | Profil |
|---|---|
| K > 0 | elliptique oblate (grand axe // **Oz**) |
| K = 0 | sphérique |
| -1 < K < 0 | elliptique prolate (grand axe // **Oz**) |
| K = -1 | parabolique |
| K < -1 | hyperbolique |

Note : Les valeurs des rayons R, K et les coefficients $\alpha$ sont à définir. Il s'agira de surfaces polynomiales asphériques, notamment le plus souvent de type hyperbolique.

La fonction z(r) définie ci-dessus suppose que l'origine **I** du repère est située au point de la surface asphérique qui coupe l'axe **Iz.** Sinon, la fonction ci-dessus est à affecter d'une constante correspondant au décalage, selon l'axe **Iz**, entre l'origine **I** du repère et le point de la surface asphérique qui coupe l'axe **Iz**.

La surface optique primaire arrière **18** et la surface optique primaire avant **20** définissent, comme une lentille optique, le jeu primaire de propriétés optiques.

Au sens du présent texte un jeu particulier de propriétés optiques peut notamment comprendre un ou plusieurs parmi, ou être constitué par :

○ une vergence particulière,
○ un niveau d'aberration sphérique (longitudinale et/ou transversale) particulier, ou une absence d'aberration sphérique,
○ un astigmatisme particulier ou une absence d'astigmatisme,
○ une défocalisation particulière,
○ un coma particulier,
○ un trefoil particulier,
○ un tetrafoil particulier.

Ainsi, deux jeux particuliers de propriétés optiques peuvent différer par une ou plusieurs parmi une vergence, un niveau d'aberration sphérique, une absence d'aberration sphérique, etc....

Une vergence particulière peut être associée à un point focal associé sur un axe principal de l'implant. Dans le cas d'un dioptre présentant des aberrations, notamment des aberrations sphériques, le point focal peut être représenté par le foyer paraxial, défini par l'approximation de l'optique géométrique, ou, de préférence, par le « meilleur foyer », qui correspond au plan pour lequel la tache de focalisation est la moins diffuse.

Dans un exemple, un jeu de propriété optique est constitué par : une vergence particulière, un niveau d'aberration sphérique (longitudinale et/ou transversale) particulier, ou une absence d'aberration sphérique, et un astigmatisme particulier ou une absence d'astigmatisme.

Dans un exemple, ce jeu primaire de propriétés optiques est un jeu particuliers de propriétés optiques qui est par exemple identifié par une vergence primaire, que l'on peut associer à un foyer primaire **F1** auquel convergent tous les rayons qui traversent l'implant **10** après avoir impacté l'implant selon une direction parallèle à l'axe optique, donc parallèle à l'axe primaire **O1**, en ayant traversé la première surface optique primaire **18**. Le foyer primaire **F1** est de préférence situé sur l'axe optique de l'implant, donc dans l'exemple sur l'axe primaire **01**. On se situe là dans le cas d'un implant sans aberrations, notamment sans aberrations sphériques. Dans le cas de la présence d'aberrations, notamment d'aberrations sphériques, le foyer primaire est remplacé par une zone de focalisation qui peut être représentée par le foyer paraxial, défini par l'approximation de l'optique géométrique, ou, de préférence, par le « meilleur foyer », qui correspond au plan pour lequel la tache de focalisation est la moins diffuse. Le jeu primaire de propriétés optiques est par exemple identifié par la vergence primaire et par un niveau primaire d'aberration sphérique, notamment longitudinale.

Par exemple, les surfaces optiques primaires arrière et avant sont choisies pour définir une correction optique permettant de corriger la vision de loin. Cette correction optique peut correspondre à une correction de 0 à 30 dioptries.

De manière générale, les surfaces optiques primaires arrière et avant ne sont pas nécessairement de même géométrie. Elles ont cependant de préférence toutes les deux le même axe de symétrie de révolution correspondant à l'axe primaire **O1**.

Afin d'obtenir un implant à propriétés optique complexes, l'une des deux faces, en l'occurrence la face arrière **14,** comporte au moins un dispositif réfractif auxiliaire **22**.

Le dispositif réfractif auxiliaire **22** comporte au moins un élément de surface optique secondaire **24** qui s'étend sur une surface de base secondaire courbe définissant, par réfraction, un jeu secondaire de propriétés optiques qui diffère, par au moins une propriété optique, du jeu primaire de propriétés optiques. Le jeu secondaire de propriétés optiques est bien entendu lié non seulement à l'élément de surface optique secondaire **24,** mais aussi à un élément de surface correspondant de la deuxième face du corps optique de l'implant, ici la face avant **16**. Dans l'exemple illustré, le jeu secondaire de propriétés optiques résulte donc de la géométrie de la surface de base secondaire et de la géométrie de la surface de base primaire avant de la surface optique avant **20**. Dans l'exemple, illustré, la face avant **20** est constituée, sur toute sa superficie utile à la vision, d'une unique surface optique qui suit, sur toute cette superficie utile à la vision, la surface de base primaire avant courbe. Ce jeu secondaire de propriétés optiques affecte donc tous les rayons lumineux qui traversent le corps optique **12** de l'implant **10** en traversant la surface optique secondaire **24**.

Le jeu secondaire de propriétés optiques est par exemple identifié par une vergence secondaire, que l'on peut associer à un foyer secondaire **F2** auquel convergent tous les rayons qui traversent l'implant **10** après avoir impacté l'implant selon une direction parallèle à l'axe optique, donc parallèle à l'axe primaire **O1**, en ayant traversé la surface optique secondaire **24**. Le foyer secondaire **F2** est de préférence situé sur l'axe optique de l'implant, donc dans l'exemple sur l'axe primaire **01**. Dans le cas de la présence d'aberrations, notamment d'aberrations sphériques, le foyer secondaire est remplacé par une zone de focalisation qui peut être représentée par le foyer paraxial, défini par l'approximation de l'optique géométrique, ou, de préférence, par le « meilleur foyer », qui correspond au plan pour lequel la tache de focalisation est la moins diffuse. Le jeu secondaire de propriétés optiques est alors par exemple identifié par la vergence secondaire et par un niveau secondaire d'aberration sphérique, notamment longitudinale.

Dans un exemple de réalisation, les jeux primaire et secondaire se distinguent l'un de l'autre par une vergence différente. La vergence primaire est donc différente de la vergence secondaire. Cependant, les jeux primaires et secondaires peuvent se distinguer l'un de l'autre par leurs niveaux d'aberration sphérique différents, ou par la présence d'aberrations sphériques pour l'un et pas pour l'autre, ceci éventuelle en plus d'une différence de vergence, ou en l'absence de différence de vergence.

Généralement, ce dispositif réfractif auxiliaire forme une excroissance ou une dépression dans la face arrière **14** par rapport à la surface primaire de base arrière de la surface optique primaire arrière **18**. Dans l'exemple illustré, le dispositif réfractif auxiliaire **22** est formé par une excroissance dans la face arrière par rapport à la surface primaire de base arrière. En d'autres termes, le dispositif réfractif auxiliaire est formé en relief.

Tout comme la surface de base primaire, la surface de base secondaire, sur laquelle s'étend cette surface optique secondaire, est de préférence une surface de révolution, c'est-à-dire ayant un axe de symétrie de révolution. De préférence, l'axe de symétrie de révolution de la surface de base secondaire est confondu avec l'axe optique **O1**, c'est-à-dire avec l'axe primaire de symétrie de révolution de la surface de base primaire arrière. Cette surface de base secondaire **24** peut être une surface sphérique mais est de préférence une surface asphérique, par exemple du type décrit plus haut, avec toutefois une valeur de rayon de courbure R et/ou une valeur de conicité K différentes de celles de la surface de base primaire arrière.

Dans l'exemple illustré, la surface optique primaire **18** et la surface optique secondaire **24** sont toutes les deux des surfaces convexes.

Dans l'exemple illustré, les surfaces optiques primaire et secondaire **18, 20** ont une disposition coaxiale, avec l'une parmi la surface optique primaire et la surface optique secondaire qui s'étend radialement vers l'extérieur autour de l'autre par rapport à l'axe primaire, donc qui entoure l'autre. Plus précisément, dans l'exemple illustré à la **Fig. 1,** le raccordement entre la surface optique primaire **18** et la surface optique secondaire **24** se fait selon un cercle dont l'axe est l'axe primaire **O1**.

De manière arbitraire, on considère dans les exemples illustrés que la surface optique primaire est une surface périphérique qui s'étend autour de la surface optique secondaire centrale par rapport à l'axe primaire **O1**. On a alors une surface optique secondaire **24** présentant un contour externe circulaire et une surface optique primaire **18** présentant un contour interne circulaire. Cependant, l'inverse est possible.

Les **Figs. 1** à **3** illustrent un implant intraoculaire selon l'art antérieur. Dans cet exemple, la surface optique primaire **18** et la surface optique secondaire **24** se raccordent l'une à l'autre, sans surface de transition, le long d'une ligne de raccordement **25** qui, dans l'exemple illustré à la **Fig. 1** un, est un cercle dont l'axe est l'axe primaire **O1**. Comme on le voit sur la **Fig. 2** et sur la **Fig. 3,** un tel raccordement, sans surface de transition à géométrie maîtrisée, génère une rupture de pente dans la face arrière **14,** cette rupture de pente étant localisée au niveau de la ligne de raccordement **25**. Des rayons lumineux qui impactent la face arrière **14** de l'implant **10** auront une direction non maîtrisée et seront susceptibles d'être focalisé au travers de l'implant en des points non maîtrisés de l'axe optique **O1**. On note que la ligne de raccordement **25** peut en réalité être une zone de raccordement dont la géométrie ne correspond pas exactement à une ligne, du fait tout simplement des contraintes de fabrication. Dans ce cas, la géométrie de la zone de raccordement peut avoir une certaine dimension selon l'axe **Ir,** dimension qui peut dépendre des techniques de fabrication de l'implant. Cependant, dans les techniques antérieures, l'objectif était de minimiser une telle zone de raccordement dans le but de minimiser les imperfections optiques qu'elle apporte.

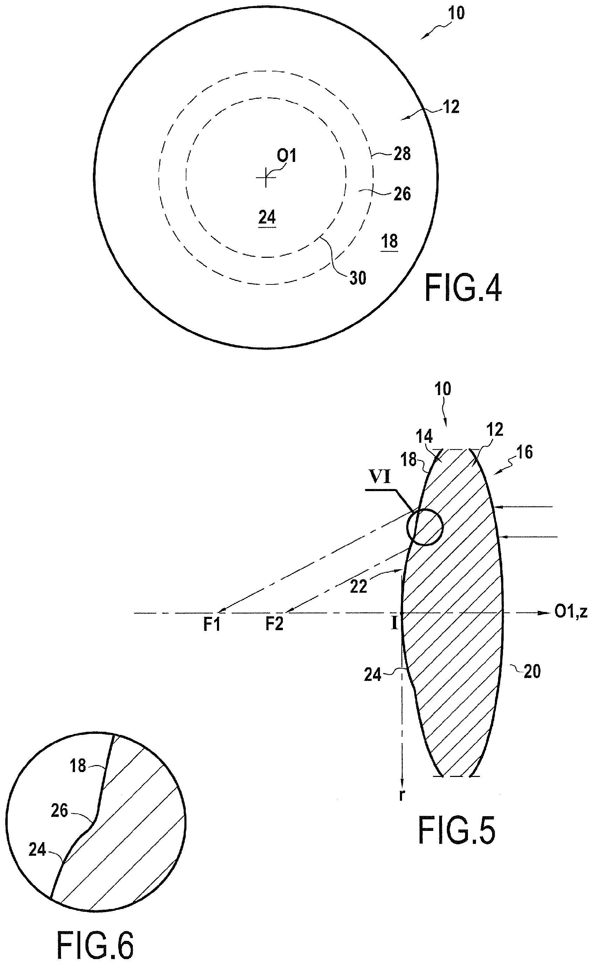

Au contraire, un implant intraoculaire optique tel qu'illustré sur les **Figs. 4** à **6** comporte, sur la première face **14,** au moins sur un secteur angulaire de transition autour de l'axe primaire **O1**, une surface de transition **26** qui s'étend, selon une direction radiale par rapport à l'axe primaire, entre la première surface optique primaire **18** et la surface optique secondaire **24**.

De préférence, comme dans l'exemple illustré, le secteur angulaire de transition sur lequel s'étend la surface de transition est de 360 degrés d'angle autour de l'axe primaire **O1**.

La présence de cette surface de transition **26** se traduit par le fait que, dans un plan de coupe considéré contenant l'axe primaire, le plan de coupe considéré coupe respectivement la première surface optique primaire **18,** la surface de transition **26** et la surface optique secondaire **24** respectivement selon un tronçon primaire **118,** un tronçon de transition **126** et un tronçon secondaire **124**. Ces tronçons **118, 126, 124** sont donc la trace respective des surfaces correspondantes **18, 26, 24** dans le plan de coupe considéré. Les tronçons **118, 126, 124** se raccordent dans cet ordre respectivement en un point de transition primaire **128** et un point de transition secondaire **130**. Le point de transition primaire **128** correspond donc au point commun entre le tronçon primaire **118** et le tronçon de transition **126**. De même, le point de transition secondaire **130** correspond donc au point commun entre le tronçon secondaire **124** et le tronçon de transition **126**.

Dans la mesure où la surface de transition **26** s'étend au moins sur un secteur angulaire de transition, ayant bien évidemment une étendue angulaire non nulle autour de l'axe primaire **O1**, le point de transition primaire **128,** dans chaque plan de coupe considéré contenant l'axe primaire **O1** et compris dans le secteur angulaire de transition, est agencé sur une ligne de transition primaire **28** dont l'étendue angulaire autour de l'axe primaire **O1** correspond au secteur angulaire. Comme on le verra plus loin, dans l'exemple illustré, cette ligne de transition primaire **28** est, dans l'exemple illustré, un arc de cercle, plus précisément dans le cas illustré un arc de cercle sur 360° autour de l'axe primaire **O1**, donc un cercle complet. De manière similaire, le point de transition secondaire **130,** dans chaque plan de coupe considéré contenant l'axe primaire **O1** et compris dans le secteur angulaire de transition, est agencé sur une ligne de transition secondaire **30** dont l'étendue angulaire autour de l'axe primaire **O1** correspond au secteur angulaire de transition. Comme on le verra plus loin, dans l'exemple illustré, cette ligne de transition secondaire **30** est un arc de cercle, plus précisément dans le cas illustré un arc de cercle sur 360° autour de l'axe **O1**, donc un cercle complet.

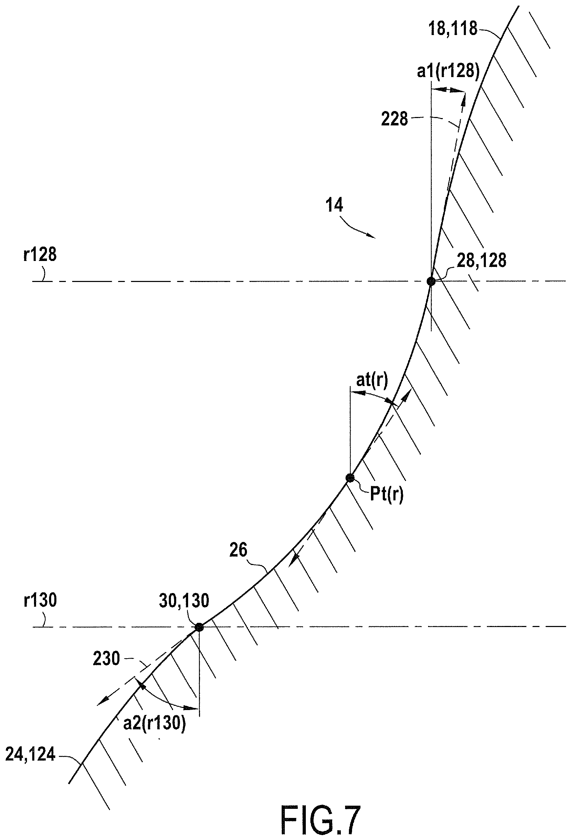

Dans l'exemple illustré, la ligne de transition primaire **28** et la ligne de transition secondaire **30** sont donc deux cercles coaxiaux ayant comme axe de symétrie l'axe primaire **O1**. Dans l'exemple illustré, la ligne de transition primaire **28,** circulaire, présente un diamètre supérieur à la ligne de transition secondaire **30** circulaire. On remarque cependant sur la Fig. 7 que chacune de la ligne de transition primaire **28** et de la ligne de transition secondaire **30** est agencée dans son propre plan respectif, les deux plans étant décalés selon la direction de l'axe **Iz**.

Dans le plan de coupe considéré contenant l'axe primaire **O1**, en l'occurrence le plan de coupe de la **Fig. 7**, le tronçon primaire **118** présente, au point de transition primaire **128**, une tangente primaire **228** ayant une orientation primaire **a1(r128),** et au point de transition secondaire **130**, le tronçon secondaire **124** présente une tangente secondaire **230** ayant une orientation secondaire **a2(r130)**. L'orientation primaire **a1(r128)** de la tangente primaire **228** correspond à l'angle aigu formé par la tangente primaire **228,** avec un axe de référence, par exemple dans l'exemple illustré avec l'axe **Ir** perpendiculaire à l'axe primaire **O1**. De même, l'orientation secondaire **a2(r130)** de la tangente secondaire **230** correspond à l'angle aigu formé par la tangente secondaire **230** avec le même axe de référence, par exemple dans l'exemple illustré avec l'axe **Ir** perpendiculaire à l'axe primaire **O1.**

Comme on l'a vu plus haut, le tronçon primaire **118** et le tronçon secondaire **124** sont des tronçons de courbe qui correspondent respectivement à l'intersection de la surface optique primaire **18** et de la surface optique secondaire **24** avec le plan de coupe considéré. Dans ce plan de coupe, le tronçon primaire **118** peut être décrit, dans un repère orthogonal et normé, par exemple le repère (**I, Ir, Iz**) décrit ci-dessus, par une fonction z1(r) et le tronçon secondaire peut être décrit par une fonction z2(r). Dans ce cas, sauf en des points très particuliers pour lesquels la fonction z1(r) et/ou z2(r) ne serait pas dérivables par rapport à la variable r, l'orientation de la tangente en un point du tronçon primaire **118** ou du tronçon secondaire est liée à la valeur, en ce point, de la dérivée z1'(r) ou z2'(r) par rapport à l'écartement radial r. La valeur de la dérivée d'une fonction en un point est aussi appelée pente ou coefficient directeur de la tangente à la courbe de cette fonction en ce point. Notamment, dans le repère mentionné ci-dessus, on a :

$$a1(r) = Arctan\,(z1'(r))$$

$$a2(r) = Arctan\,(z2'(r))$$

Toujours dans le même repère, on peut définir que le point de transition primaire **128** possède comme coordonnées (r128, z1(r128)), et que le point de transition secondaire **130** possède comme coordonnées (r130, z2(r130)). Ainsi :

$$\text{l'orientation primaire est la valeur } a1\,(r128) = Arctan\,(z1'(r128))$$

$$\text{l'orientation secondaire est la valeur } a2\,(r130) = Arctan\,(z2'(r130))$$

Selon un aspect de l'invention, dans tout plan de coupe considéré contenant l'axe primaire **O1** et compris dans un secteur angulaire de transition autour de l'axe primaire **O1**, la surface de transition **26** présente, comme illustré à la **Fig. 7,** en tout point donné **Pt(r)** du tronçon de transition **126,** une tangente locale de transition au tronçon de transition dont l'orientation **at(r)** est comprise dans la plage angulaire allant de l'orientation primaire **a1(r128)** à l'orientation secondaire **(a2(r130)).** L'orientation **at(r)** de la tangente locale de transition correspond à l'angle aigu formé par la tangente locale de transition avec le même axe de référence que celui choisi précédemment, par exemple dans l'exemple illustré avec l'axe **Ir** perpendiculaire à l'axe primaire **O1**.

Dans ce texte, on désigne par surface de transition toute surface optique de l'implant comprise entre la surface optique primaire et la surface optique secondaire et respectant la caractéristique ci-dessus, ainsi qu'éventuellement une ou plusieurs des dispositions optionnelles décrites ci-dessous. L'implant **10** peut présenter, en plus, des surfaces de raccordement ne respectant pas cette disposition.

De même, on désigne par secteur angulaire de transition le secteur angulaire autour de l'axe primaire **O1** dans lequel la surface de transition respecte la caractéristique ci-dessus.

On notera que, dans un repère orthogonal et normé, en supposant que le tronçon de transition **126** soit décrit par la fonction zt(r), cela se traduit par le fait que la pente zt'(r) de la tangente locale de transition, en tout point **Pt(r)** du tronçon de transition **126,** est comprise dans l'intervalle allant de la pente primaire z1'(r128) à la pente secondaire z2'(r130).

Grâce à cette disposition, tout rayon lumineux qui traverse l'implant **10** après l'avoir impacté dans le plan de coupe considéré, selon une direction parallèle à l'axe optique, donc parallèle à l'axe primaire **O1**, en ayant traversé la surface optique de transition **26**, ressort de l'implant en convergeant vers un point de l'axe optique **O1** qui, dans le cas d'un implant sans aberration sphérique, est compris dans l'intervalle allant du foyer primaire **F1** au foyer secondaire **F2**. Dans le cas de surfaces optiques primaire et/ou secondaire présentant des aberrations, notamment des aberrations sphériques, ce rayon lumineux convergera vers un point compris dans l'intervalle défini par les deux zones de focalisation. Cette caractéristique est vraie pour tous les points de la surface de transition dans ledit secteur angulaire de transition. Ainsi, la surface de transition n'est pas le siège d'une déperdition lumineuse susceptible de créer des artefacts qui diminuent la performance de l'implant, ou qui le rendent plus difficile à accepter par le patient. Ce point de convergence n'est pas nécessairement le même pour tous les rayons qui impactent la surface optique de transition **26** dans le plan de coupe considéré, et ce point de convergence peut varier en fonction de l'écartement radial r du point de la surface de transition impacté par le rayon lumineux.

On note que, dans tout plan de coupe considéré contenant l'axe primaire **O1** et compris dans le secteur angulaire de transition autour de l'axe primaire **O1**, la surface de transition **26** présente un tronçon de transition **126** continu, sans saut. Cela peut se traduire par le fait que la fonction zt(r), exprimée par exemple dans le repère **(I, Ir, Iz),** est une fonction continue en tout point **Pt(r)** depuis le point de transition primaire **128** jusqu'au point de transition secondaire **130.**

La surface de transition **26** peut avoir une ou plusieurs des dispositions optionnelles décrites ci-dessous, sur un secteur angulaire compris dans le secteur de transition et ayant une étendue angulaire égale ou inférieure à celle du secteur angulaire de transition mais non nulle autour de l'axe primaire **O1**.

De préférence, dans tout plan de coupe considéré contenant l'axe primaire **O1** et compris dans un secteur dudit secteur angulaire de transition autour de l'axe primaire **O1**, pour tout point **Pt(r)** donné du tronçon de transition **126,** l'orientation at(r) de la tangente locale de transition évolue, le long du tronçon de transition depuis le point de transition primaire **128** jusqu'au point de transition secondaire **130,** selon une fonction continue de l'écartement radial r du point donné, l'écartement radial étant la distance selon une direction perpendiculaire à l'axe primaire, entre le point donné et l'axe primaire. Cette évolution selon une fonction continue évite la présence de ruptures de pentes le long du tronçon de transition, donc sur l'étendue de la zone de transition. En d'autres termes, la pente de la tangente locale de transition évolue elle aussi selon une fonction continue de l'écartement radial r du point donné, le long du tronçon de transition depuis le point de transition primaire **128** jusqu'au point de transition secondaire **130.** Par l'absence de rupture de pente, on limite encore les artefacts, et on obtient une meilleure acceptabilité de l'implant.

De préférence, comme illustré à la **Fig. 7,** dans tout plan de coupe considéré contenant l'axe primaire **O1** et compris dans un secteur dudit secteur angulaire de transition autour de l'axe primaire **O1**, l'orientation at(r128) de la tangente locale de transition au point de transition primaire **128** est égale à l'orientation primaire **a1(r128).** En d'autres termes, les tangentes de part et d'autre du point de transition primaire, respectivement au tronçon primaire et au tronçon de transition, sont alignées. Autrement dit, la pente zt'(r128) de la tangente locale de transition au point de transition primaire **128** est égale à la pente z1'(r128) de la tangente au tronçon primaire **118** au point de transition primaire (**128**). Il n'y a donc pas de rupture de pente entre la surface optique primaire et la surface de transition en ce point. Cela limite encore les artefacts, et on obtient une meilleure acceptabilité de l'implant.

De préférence, comme illustré à la **Fig. 7,** dans tout plan de coupe considéré contenant l'axe primaire **O1** et compris dans un secteur dudit secteur angulaire de transition autour de l'axe primaire **O1**, l'orientation at(r130) de la tangente locale de transition au point de transition secondaire **130** est égale à l'orientation secondaire **a2(r130).** En d'autres termes, les tangentes de part et d'autre du point de transition secondaire, respectivement au tronçon secondaire et au tronçon de transition, sont alignées. Autrement dit, la pente zt'(r130) de la tangente locale de transition au point de transition secondaire **130** est égale à la pente z2'(r130) de la tangente au tronçon secondaire **124** au point de transition secondaire **130**. Il n'y a donc pas de rupture de pente entre la surface optique secondaire et la surface de transition en ce point. Cela limite encore les artefacts, et on obtient une meilleure acceptabilité de l'implant.

De manière générale, pour assurer la continuité de la variation de la pente dans tout plan de coupe considéré

contenant l'axe primaire (O1) et compris dans un secteur dudit secteur angulaire de transition autour de l'axe primaire, la surface de transition présente, en tout point donné du tronçon de transition, une dérivée de la pente de la tangente locale de transition qui varie, depuis le point de transition primaire jusqu'au point de transition secondaire, selon une fonction continue, par exemple polynomiale, de l'écartement radial dans un repère orthogonal et normé du plan de coupe considéré, par exemple le repère (**I**, **Ir, Iz**). Ici, on dérive la fonction zt'(r) exprimant la pente par rapport à l'écartement radial :

$$zt''(r) = d/dr\,[z't(r)]$$

Dans certains modes de réalisation on peut prévoir que, dans tout plan de coupe considéré contenant l'axe primaire (O1) et compris dans un secteur dudit secteur angulaire de transition autour de l'axe primaire, le tronçon de transition est un tronçon de courbe conique, par exemple un tronçon de courbe parabolique.

Dans un exemple de réalisation, la surface de transition **26** est conçue de telle sorte, dans tout plan de coupe considéré contenant l'axe primaire **O1** et compris dans un secteur dudit secteur angulaire de transition autour de l'axe primaire **O1**, la surface de transition **26** présente, en tout point donné **Pt(r)** du tronçon de transition **126** depuis le point de transition primaire jusqu'au point de transition secondaire, une pente zt'(r) de la tangente locale de transition qui varie selon une fonction linéaire dans un repère orthogonal et normé du plan de coupe considéré. En d'autres termes, dans le repère (**I**, **Ir**, **Iz**), la fonction zt'(r) qui décrit la pente de la tangente locale de transition, s'écrit sous la forme :

$$zt'(r) = (c * r) + d.$$

Il en découle, par intégration de cette fonction continue, que le tronçon de transition présente, dans ce repère, une équation sous la forme :

$$zt(r) = 0.5 * c * r\char`\^2 + d * r + e$$

Plus préférentiellement, dans un plan de coupe considéré, on construit le tronçon de transition **126** de la manière suivante. On choisit d'abord le point de transition primaire et le point de transition secondaire en fixant leur écartement respectif **r128** et **r130** par rapport à l'axe primaire. Pour le point de transition primaire, on détermine la pente primaire du tronçon primaire qui est la valeur z1'(r128). Pour le point de transition secondaire, on détermine la pente secondaire du tronçon secondaire qui est la valeur z2'(r130).

On utilise alors la caractéristique optionnelle selon laquelle, dans une variante préférée de l'invention, la pente de la tangente locale de transition en ces deux points est respectivement égale à la pente primaire et à la pente secondaire.

$$zt'(r128) = z1'(r128)$$

et

$$zt'(r130) = z2'(r130)$$

On calcule alors le paramètre c de linéarité de la pente de la tangente locale de transition, et le paramètre d, selon les deux formules suivantes :

$$c = [z1'(r128) - z2'(r130)] / [r128 - r130]$$

$$d = z2'(r130) - c * r130$$

Le paramètre e est déterminé pour que l'on ait la relation

$$zt(r130) = z2(r130)$$

grâce à laquelle on s'assure que le tronçon de transition **126** et le tronçon secondaire **124** se rejoignent au point de transition secondaire **130**. Par le mode de calcul ci-dessus, cela assure aussi que le tronçon de transition **126** et le tronçon primaire **118** se rejoignent au point de transition primaire **128**.

En ayant déterminé les paramètres c, d et e, on connaît le profil à donner au tronçon de transition **126**.

Lorsque les surfaces optiques primaire **18** et secondaire **24** sont des surfaces de révolution, notamment autour du même axe de symétrie de révolution **O1**, la surface de transition s'étend elle aussi sur une surface de révolution ayant comme axe de révolution l'axe primaire **O1**, tout du moins dans le secteur angulaire de transition. La surface de transition **26** est alors obtenue par rotation du tronçon de transition **126** sur l'étendue angulaire du secteur angulaire de transition. Bien entendu, dans la plupart des cas, on veillera à ce que le secteur de transition s'étendent sur 360° autour de l'axe primaire **O1**, auquel cas, la surface de transition sera une surface de révolution sur 360° autour de l'axe **O1**.

Dans l'exemple illustré, la surface optique primaire **18** et la surface optique secondaire **24** sont toutes les deux des surfaces convexes, tandis que la surface de transition **26** est une surface concave.

Dans les exemples et variantes décrites ci-dessus, on a considéré un implant ayant uniquement, sur une même première face, une surface optique primaire et une surface optique secondaire avec donc une surface de transition selon l'invention entre les deux.

Cependant, l'invention peut aussi être avantageusement mise en oeuvre avec plusieurs dispositifs réfractifs auxiliaires distincts. Ainsi, un dispositif réfractif auxiliaire supplémentaire peut comporter alors un élément de surface optique tertiaire qui s'étend selon une surface de base tertiaire courbe définissant, par réfraction, un jeu tertiaire de propriétés optiques, distinct au moins du jeu secondaire de propriété optique. De la sorte, on peut par exemple réaliser un implant tri-focal.

Tout comme les surfaces de base primaire ou secondaire, cette surface de base tertiaire est aussi de préférence, une surface sphérique ou asphérique, de révolution, de préférence avec comme axe de révolution aussi l'axe primaire. De préférence, la surface optique tertiaire est elle aussi une surface de contour, interne et/ou externe, circulaire autour de l'axe primaire **O1**. Par exemple, la surface optique secondaire est une surface qui s'étend autour de la surface optique secondaire par rapport à l'axe primaire **O1**. On a alors une surface optique tertiaire qui présente un contour externe circulaire et une surface optique secondaire présentant un contour interne circulaire. Cependant, l'inverse est possible.

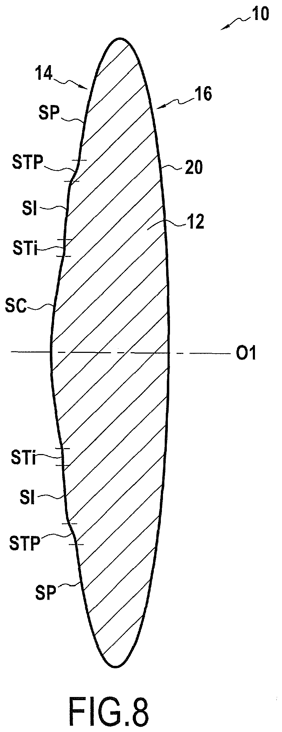

Ainsi, l'invention peut être mise en œuvre pour plusieurs zones de transition sur une même face d'un même implant. On a illustré sur la **Fig. 8** une vue schématique en coupe d'un implant ayant une surface optique périphérique **SP,** une surface optique intermédiaire **SI** et une surface optique centrale **SC**. Ces trois surfaces optiques sont dans le cas illustré des surfaces de révolution coaxiales autour d'un axe primaire **O1** qui correspond dans ce cas à l'axe optique de l'implant **10.**

Entre la surface périphérique **SP** et la surface intermédiaire **SI,** on a prévu une surface de transition périphérique **STP** conforme aux enseignements de l'invention. Pour cette surface de transition périphérique on considère que l'une parmi la surface périphérique **SP** et la surface intermédiaire **SI** est une surface optique primaire, et donc que l'autre est une surface optique secondaire.

Entre la surface intermédiaire **SI** et la surface centrale **SC,** on a prévu une surface de transition intermédiaire **STI** conforme aux enseignements de l'invention. Pour cette surface de transition intermédiaire **STI** on considère que l'une parmi la surface centrale **SC** et la surface intermédiaire **SI** est une surface optique primaire, et donc que l'autre est une surface optique secondaire.

En pratique, la surface de transition doit avoir une dimension réduite selon la direction radiale. De manière générale, la surface de transition **26** est de préférence comprise entre un cercle de transition primaire et un cercle de transition secondaire qui présentent une différence de rayon de 0.5 millimètre ou moins, de préférence 0.15 millimètre ou moins. La différence de rayon est par exemple comprise dans la gamme allant de 0.05 à 0.5 millimètres, de préférence comprise dans la gamme allant de 0.05 à 0.15 millimètres. Dans le cas de surfaces optique primaire et secondaire de révolution, le cercle de transition primaire 28 et le cercle de transition secondaire 30, qui délimitent la surface de transition, présentent ainsi de préférence une différence de rayon (r128 - r130) de 0.5 millimètre ou moins, de préférence 0.15 millimètre ou moins. La différence de rayon est par exemple comprise dans la gamme allant de 0.05 à 0.5 millimètres, de préférence compris comprise dans la gamme allant de 0.05 à 0.15 millimètres.

L'invention n'est pas limitée aux exemples décrits et représentés car diverses modifications peuvent y être apportées sans sortir de son cadre.

**Revendications**

1. Implant optique intraoculaire (**10**) :

   - du type comportant un corps optique réfractif (**12**) présentant deux faces, respectivement arrière (**14**) et avant (**16**), comportant chacune respectivement une surface optique primaire, respectivement arrière (**18**) et avant (**20**), qui s'étendent chacune respectivement sur une surface de base primaire courbe, respectivement arrière et avant, les surfaces de bases primaires courbes définissant, par réfraction, un jeu primaire de propriétés optiques ;
   - du type dans lequel, parmi les faces arrière ou avant, au moins une première face (**14**, **16**), comporte une première surface optique primaire (**18**) s'étendant sur une première surface de base primaire courbe, et au moins une surface optique secondaire (**24**) qui s'étend sur une surface de base secondaire courbe définissant, par réfraction, une jeu secondaire de propriétés optiques;
   - du type dans lequel la première surface de base primaire est une surface de révolution autour d'un axe primaire (**O1**) ;
   - et du type dans lequel la surface de base secondaire est une surface de révolution ;

   **caractérisé en ce que** :

   - la première face (**14**) comporte, au moins sur un secteur angulaire de transition autour de l'axe primaire (**O1**), une surface de transition (**26**) qui s'étend, selon une direction radiale par rapport à l'axe primaire, entre la première surface optique primaire (**18**) et la surface optique secondaire (**24**) ;
   - **en ce qu'**un plan de coupe considéré, contenant l'axe primaire (**O1**), coupe respectivement la première surface optique primaire (**18**), la surface de transition (**26**) et la surface optique secondaire (**24**) respectivement selon un tronçon primaire (**118**), un tronçon de transition (**126**) et un tronçon secondaire (**124**), les tronçons se raccordant dans cet ordre respectivement en un point de transition primaire (**128**) et un point de transition secondaire (**130**) ;
   - **en ce que** dans le plan de coupe considéré, le tronçon primaire (**118**) présente, au point de transition primaire (**128**), une tangente primaire (**228**) ayant une orientation primaire (**a1(r128)),** et au point de transition secondaire (**130**), le tronçon secondaire (**124**) présente une tangente secondaire (**230**) ayant une orientation secondaire (**a2(r130))** ;
   - et **en ce que**, dans tout plan de coupe considéré contenant l'axe primaire (**O1**) et compris dans le secteur angulaire de transition autour de l'axe primaire, la surface de transition (**26**) présente, en tout point donné du tronçon de transition (**126**), une tangente locale de transition au tronçon de transition dont l'orientation (**at(r)**) est comprise dans la plage angulaire allant de l'orientation primaire (**a1(r128)**) à l'orientation secondaire (**a2(r130)**).

2. Implant optique intraoculaire selon la revendication **1, caractérisé en ce que**, dans tout plan de coupe considéré contenant l'axe primaire (**O1**) et compris dans un secteur dudit secteur angulaire de transition autour de l'axe primaire, pour tout point donné (**Pt(r)**) du tronçon de transition (**126**), l'orientation (**at(r)**) de la tangente locale de transition évolue, le long du tronçon de transition (**126**) depuis le point de transition primaire (**128**) jusqu'au point de transition secondaire (**130**), selon une fonction continue de l'écartement radial (r) du point donné (**Pt(r)**), l'écartement radial étant la distance, selon une direction perpendiculaire à l'axe primaire, entre le point donné et l'axe primaire.

3. Implant optique intraoculaire selon l'une quelconque des revendications précédentes, **caractérisé en ce que** dans tout plan de coupe considéré contenant l'axe primaire (**O1**) et compris dans un secteur dudit secteur angulaire de transition autour de l'axe primaire, l'orientation (**at(r128)**) de la tangente locale de transition au point de transition primaire (**128**) est égale à l'orientation primaire (**a1(r128)**).

4. Implant optique intraoculaire selon l'une quelconque des revendications précédentes, **caractérisé en ce que**, dans tout plan de coupe considéré contenant l'axe primaire (**O1**) et compris dans un secteur dudit secteur angulaire de transition autour de l'axe primaire, l'orientation de la tangente locale de transition au point de transition secondaire (**at(r130)**) est égale à l'orientation secondaire (**a2(r130)**).

5. Implant optique intraoculaire selon l'une quelconque des revendications précédentes, **caractérisé en ce que**, dans tout plan de coupe considéré contenant l'axe primaire (**O1**) et compris dans un secteur dudit secteur angulaire de transition autour de l'axe primaire, la surface de transition (**26**) présente, en tout point donné (**Pt(r)**) du tronçon de transition (**126**) depuis le point de transition primaire (**128**) jusqu'au point de transition secondaire (**130**), une dérivée (zt''(r)) de la pente de la tangente locale de transition qui varie selon une fonction continue, de préférence polynomiale, de l'écartement radial (**r**) dans un repère orthogonal et normé du plan de coupe considéré.

6. Implant optique intraoculaire selon l'une quelconque des revendications précédentes, **caractérisé en ce que**, dans tout plan de coupe considéré contenant l'axe primaire (**O1**) et compris dans un secteur dudit secteur angulaire de transition autour de l'axe primaire, la surface de transition (**26**) présente, en tout point donné (Pt(r)) du tronçon de transition (**126**) depuis le point de transition primaire (**128**) jusqu'au point de transition secondaire (**130**), une pente (zt'(r)) de la tangente locale de transition qui varie selon une fonction linéaire de l'écartement radial (r) dans un repère orthogonal et normé du plan de coupe considéré.

7. Implant optique intraoculaire selon l'une quelconque des revendications précédentes, **caractérisé en ce que**, dans tout plan de coupe considéré contenant l'axe primaire (**O1**) et compris dans un secteur dudit secteur angulaire de transition autour de l'axe primaire, le tronçon de transition (**126**) est un tronçon de courbe conique.

8. Implant optique intraoculaire selon l'une quelconque des revendications précédentes, **caractérisé en ce que**, dans le secteur angulaire de transition, la surface de transition (**26**) s'étend sur une surface de révolution ayant comme axe de révolution l'axe primaire (**O1**).

9. Implant optique intraoculaire selon l'une quelconque des revendications précédentes, **caractérisé en ce que** le secteur angulaire de transition s'étend sur 360 degrés d'angle autour de l'axe primaire (**O1**).

10. Implant optique intraoculaire selon l'une quelconque des revendications précédentes, **caractérisé en ce que** l'une parmi la surface optique primaire (**18**) et la surface optique secondaire (**24**) s'étend radialement vers l'extérieur autour de l'autre par rapport à l'axe primaire (**O1**).

11. Implant optique intraoculaire selon l'une quelconque des revendications précédentes, **caractérisé en ce que** l'une ou l'autre, ou les deux, parmi la surface optique primaire (**18**) et la surface optique secondaire (**20**), est une surface de révolution autour de l'axe primaire (**O1**).

12. Implant optique intraoculaire selon l'une quelconque des revendications précédentes, **caractérisé en ce que** la surface de transition (**26**) est de préférence comprise entre un cercle de transition primaire et un cercle de transition secondaire qui présentent une différence de rayon de 0.5 millimètre ou moins, de préférence 0.15 millimètre ou moins, plus préférentiellement comprise dans la gamme allant de 0.05 à 0.15 millimètres.

13. Implant optique intraoculaire selon l'une quelconque des revendications précédentes, **caractérisé en ce que** la surface de transition (**26**) est délimitée par et comprise entre un cercle de transition primaire (**28**) commun avec la surface optique primaire (**18**) et un cercle de transition secondaire (**30**) commun avec la surface optique secondaire (**24**).

14. Implant optique intraoculaire selon la revendication **13, caractérisé en ce que** le cercle de transition primaire (**28**) et le cercle de transition secondaire (**30**) présentent une différence de rayon de 0.5 millimètre ou moins, de préférence 0.15 millimètre ou moins, plus préférentiellement comprise dans la gamme allant de 0.05 à 0.15 millimètres.

15. Implant optique intraoculaire selon l'une quelconque des revendications précédentes, **caractérisé en ce que** la surface de base secondaire est une surface de révolution autour du même axe primaire (**O1**) que la première surface de base primaire.

16. Implant optique intraoculaire selon l'une quelconque des revendications précédentes, **caractérisé en ce que** la première surface optique primaire (**18**) et la surface optique secondaire (**24**) sont toutes les deux des surfaces convexes, tandis que la surface de transition (**26**) est une surface concave.

**Patentansprüche**

1. Intraokulares optisches Implantat (10):

    - des Typs, der einen refraktiven optischen Körper (12) umfasst, der zwei Seiten, jeweils eine hintere (14) und eine vordere (16), aufweist, die jeweils eine primäre optische Oberfläche, jeweils eine hintere (18) und eine vordere (20), aufweisen, die sich jeweils auf einer gekrümmten primären Grundoberfläche, jeweils einer hinteren und einer vorderen, erstrecken, wobei die gekrümmten primären Grundoberflächen durch Brechung einen primären Satz optischer Eigenschaften definieren,

- des Typs, bei dem unter der hinteren oder der vorderen Seite mindestens eine erste Seite (14, 16) mindestens eine erste primäre optische Oberfläche (18), die sich auf einer ersten gekrümmten primären Grundoberfläche erstreckt, und mindestens eine sekundäre optische Oberfläche (24) aufweist, die sich auf einer gekrümmten sekundären Grundoberfläche erstreckt, die durch Brechung einen sekundären Satz optischer Eigenschaften definiert,

- des Typs, bei dem die erste gekrümmte primäre Grundoberfläche eine Rotationsfläche um eine primäre Achse (O1) aufweist,

- und des Typs, bei dem die sekundäre Grundoberfläche eine Rotationsfläche ist,

**dadurch gekennzeichnet, dass**:

- die erste Seite (14) mindestens auf einem Übergangswinkelbereich um die primäre Achse (O1) eine Übergangsoberfläche (26) aufweist, die sich in radialer Richtung in Bezug auf die primäre Achse zwischen der ersten primären optischen Oberfläche (18) und der sekundären optischen Oberfläche (24) erstreckt,

- dadurch, dass eine betrachtete Schnittebene, die die primäre Achse (O1) enthält, jeweils die erste primäre optische Oberfläche (18), die Übergangsoberfläche (26) und die sekundäre optische Oberfläche (24) jeweils entlang eines primären Abschnitts (118), eines Übergangsabschnitts (126) und eines sekundären Abschnitts (124) schneidet, wobei sich die Abschnitte in dieser Reihenfolge jeweils an einem primären Übergangspunkt (128) und einem sekundären Übergangspunkt (130) aneinander anschließen,

- dadurch, dass der primäre Abschnitt (118) an dem primären Übergangspunkt (128) in der betrachteten Schnittebene eine primäre Tangente (228) aufweist, die eine primäre Ausrichtung (a1(r128)) aufweist, und der sekundäre Abschnitt (124) an dem sekundären Übergangspunkt (130) eine sekundäre Tangente (230) aufweist, die eine sekundäre Ausrichtung (a2(r130)) aufweist,

- dadurch, dass in jeder betrachteten Schnittebene, die die primäre Achse (O1) enthält und die in dem Übergangswinkelbereich um die primäre Achse enthalten ist, die Übergangsoberfläche (26) an jedem gegebenen Punkt des Übergangsabschnitts (126) eine lokale Übergangstangente zu dem Übergangsabschnitt aufweist, deren Ausrichtung (at(r)) in dem Winkelbereich enthalten ist, der von der primären Ausrichtung (a1(r128)) zu der sekundären Ausrichtung (a2(r130)) geht.

2. Intraokulares optisches Implantat nach Anspruch 1, **dadurch gekennzeichnet, dass** in jeder betrachteten Schnittebene, die die primäre Achse (O1) enthält und die in einem Sektor des Übergangswinkelbereichs um die primäre Achse enthalten ist, sich die Ausrichtung (at(r)) der lokalen Übergangstangente für jeden gegebenen Punkt (Pt(r)) des Übergangsabschnitts (126) entlang des Übergangsabschnitts (126) von dem primären Übergangspunkt (128) bis zu dem sekundären Übergangspunkt (130) gemäß einer kontinuierlichen Funktion des radialen Abstands (r) von dem gegebenen Punkt (Pt(r)) entwickelt, wobei der radiale Abstand die Entfernung entlang einer Richtung, die senkrecht zu der primären Achse ist, zwischen dem gegebenen Punkt und der primären Achse ist.

3. Intraokulares optisches Implantat nach einem der vorhergehenden Ansprüche, **dadurch gekennzeichnet, dass** in jeder betrachteten Schnittebene, die die primäre Achse (O1) enthält und die in einem Sektor des Übergangswinkelbereichs um die primäre Achse enthalten ist, die Ausrichtung (at(r128)) der lokalen Übergangstangente an dem primären Übergangspunkt (128) gleich der primären Ausrichtung (a1(r128)) ist.

4. Intraokulares optisches Implantat nach einem der vorhergehenden Ansprüche, **dadurch gekennzeichnet, dass** in jeder betrachteten Schnittebene, die die primäre Achse (O1) enthält und die in einem Sektor des Übergangswinkelbereichs um die primäre Achse enthalten ist, die Ausrichtung der lokalen Übergangstangente an dem sekundären Übergangspunkt (at(r130)) gleich der sekundären Ausrichtung (a2(r130)) ist.

5. Intraokulares optisches Implantat nach einem der vorhergehenden Ansprüche, **dadurch gekennzeichnet, dass** in jeder betrachteten Schnittebene, die die primäre Achse (O1) enthält und die in einem Sektor des Übergangswinkelbereichs um die primäre Achse enthalten ist, die Übergangsoberfläche (26) an jedem gegebenen Punkt (Pt(r)) des Übergangsabschnitts (126) von dem primären Übergangspunkt (128) bis zu dem sekundären Übergangspunkt (130) eine Ableitung (zf"(r)) von der Steigung der lokalen Übergangstangente aufweist, die nach einer stetigen, vorzugsweise polynomialen Funktion des radialen Abstands (r) in einem orthogonalen und normierten Bezugssystem der betrachteten Schnittebene variiert.

6.  Intraokulares optisches Implantat nach einem der vorhergehenden Ansprüche, **dadurch gekennzeichnet, dass** in jeder betrachteten Schnittebene, die die primäre Achse (O1) enthält und die in einem Sektor des Übergangswinkelbereichs um die primäre Achse enthalten ist, die Übergangsoberfläche (26) an jedem gegebenen Punkt (Pt(r)) des Übergangsabschnitts (126) von dem primären Übergangspunkt (128) bis zu dem sekundären Übergangspunkt (130) eine Steigung (zf'(r)) der lokalen Übergangstangente aufweist, die nach einer linearen Funktion des radialen Abstands (r) in einem orthogonalen und normierten Bezugssystem der betrachteten Schnittebene variiert.

7.  Intraokulares optisches Implantat nach einem der vorhergehenden Ansprüche, **dadurch gekennzeichnet, dass** in jeder betrachteten Schnittebene, die die primäre Achse (O1) enthält und die in einem Sektor des Übergangswinkelbereichs um die primäre Achse enthalten ist, der Übergangsabschnitt (126) ein Abschnitt einer konischen Kurve ist.

8.  Intraokulares optisches Implantat nach einem der vorhergehenden Ansprüche, **dadurch gekennzeichnet, dass** sich die Übergangsoberfläche (26) in dem Übergangswinkelbereich über eine Rotationsfläche erstreckt, die als Rotationsachse die primäre Achse (O1) aufweist.

9.  Intraokulares optisches Implantat nach einem der vorhergehenden Ansprüche, **dadurch gekennzeichnet, dass** sich der Übergangswinkelbereich über einen Winkel von 360 Grad um die primäre Achse (O1) erstreckt.

10. Intraokulares optisches Implantat nach einem der vorhergehenden Ansprüche, **dadurch gekennzeichnet, dass** sich eine unter der primären optischen Oberfläche (18) und der sekundären optischen Oberfläche (24) radial nach Außen um die andere in Bezug auf die primäre Achse (O1) erstreckt.

11. Intraokulares optisches Implantat nach einem der vorhergehenden Ansprüche, **dadurch gekennzeichnet, dass** die eine oder die andere oder beide unter der primären optischen Oberfläche (18) und der sekundären optischen Oberfläche (20) eine Rotationsachse um die primäre Achse (O1) ist.

12. Intraokulares optisches Implantat nach einem der vorhergehenden Ansprüche, **dadurch gekennzeichnet, dass** die Übergangsoberfläche (26) vorzugsweise zwischen einem primären Übergangskreis und einem sekundären Übergangskreis liegt, die einen Radiusunterschied von 0,5 Millimeter oder weniger, vorzugsweise von 0,15 Millimeter oder weniger, insbesondere innerhalb des Bereichs von 0,05 bis 0,15 Millimeter, aufweisen.

13. Intraokulares optisches Implantat nach einem der vorhergehenden Ansprüche, **dadurch gekennzeichnet, dass** die Übergangsoberfläche (26) zwischen einem primären Übergangskreis (28), der mit der primären optischen Oberfläche (18) gemeinsam ist, und einem sekundären Übergangskreis (30) liegt, der mit der sekundären optischen Oberfläche (24) gemeinsam ist, und davon begrenzt ist.

14. Intraokulares optisches Implantat nach Anspruch 13, **dadurch gekennzeichnet, dass** der primäre Übergangskreis (28) und der sekundäre Übergangskreis (30) einen Radiusunterschied von 0,5 Millimeter oder weniger, vorzugsweise von 0,15 Millimeter oder weniger, insbesondere innerhalb des Bereichs von 0,05 bis 0,15 Millimeter, aufweisen.

15. Intraokulares optisches Implantat nach einem der vorhergehenden Ansprüche, **dadurch gekennzeichnet, dass** die sekundäre Grundfläche eine Rotationsfläche um die gleiche primäre Achse (O1) wie die erste primäre Grundfläche ist.

16. Intraokulares optisches Implantat nach einem der vorhergehenden Ansprüche, **dadurch gekennzeichnet, dass** die primäre optische Oberfläche (18) und die sekundäre optischen Oberfläche (24) alle beide konvexe Oberflächen sind, während die Übergangsoberfläche (26) eine konkave Oberfläche ist.

**Claims**

1.  An intraocular optical implant (10):

    - of the type including a refractive optical body (12) having two faces, respectively rear (14) and front (16) faces, each including respectively a primary optical surface, respectively rear (18) and front (20) surface, which each extend respectively on a curved primary base surface, respectively rear and front surface, the curved primary base surfaces defining, by refraction, a primary set of optical properties;

- of the type in which, among the rear or front faces, at least a first face (14, 16) includes a first primary optical surface (18) extending over a first curved primary base surface and at least a secondary optical surface (24) extending over a curved secondary base surface defining, by refraction, a secondary set of optical properties;
- of the type in which the first primary base surface is a surface of revolution around a primary axis (01);
- and of the type in which the secondary base surface is a surface of revolution;

**characterized in that**:

- the first face (14) includes, at least on an angular transition sector around the primary axis (O1), a transition surface (26) extending, along a radial direction with respect to the primary axis, between the first primary optical surface (18) and the secondary optical surface (24);
- **in that** a considered cutting plane, containing the primary axis (01), respectively cuts the first primary optical surface (18), the transition surface (26) and the secondary optical surface (24) respectively along a primary section (118), a transition section (126) and a secondary section (124), the sections connecting in this order respectively at a primary transition point (128) and a secondary transition point (130);
- **in that** in the considered cutting plane, the primary section (118) has, at the primary transition point (128), a primary tangent (228) having a primary orientation (a1(r128)), and the secondary section (124) has, at the secondary transition point (130), a secondary tangent (230) having a secondary orientation (a2(r130));
- and **in that**, in any considered cutting plane containing the primary axis (01) and comprised in the angular transition sector around the primary axis, the transition surface (26) has, at any given point of the transition section (126), a local transition tangent to the transition section whose orientation (at(r)) is comprised in the angular range from the primary orientation (a1(r128)) to the secondary orientation (a2(r130)).

2. The intraocular optical implant according to claim 1, **characterized in that**, in any considered cutting plane containing the primary axis (01) and comprised in a sector of said angular transition sector around the primary axis, for any given point (Pt(r)) of the transition section (126), the orientation (at(r)) of the local transition tangent progresses, along the transition section (126) from the primary transition point (128) to the secondary transition point (130), according to a continuous function of the radial spacing (r) of the given point (Pt(r)), the radial spacing being the distance, along a direction perpendicular to the primary axis, between the given point and the primary axis.

3. The intraocular optical implant according to any one of the preceding claims, **characterized in that** in any considered cutting plane containing the primary axis (O1) and comprised in a sector of said angular transition sector around the primary axis, the orientation (at(r128)) of the local transition tangent to the primary transition point (128) is equal to the primary orientation (a1(r128)).

4. The intraocular optical implant according to any one of the preceding claims, **characterized in that**, in any considered cutting plane containing the primary axis (O1) and comprised in a sector of said angular transition sector around the primary axis, the orientation of the local transition tangent to the secondary transition point (at(r130)) is equal to the secondary orientation (a2(r130)).

5. The intraocular optical implant according to any one of the preceding claims, **characterized in that**, in any considered cutting plane containing the primary axis (O1) and comprised in a sector of said angular transition sector around the primary axis, the transition surface (26) has, at any given point (Pt(r)) of the transition section (126) from the primary transition point (128) to the secondary transition point (130), a derivative (zt"(r)) of the slope of the local transition tangent which varies according to a continuous, preferably polynomial, function of the radial spacing (r) in an orthonormal coordinate system of the considered cutting plane.

6. The intraocular optical implant according to any one of the preceding claims, **characterized in that**, in any considered cutting plane containing the primary axis (01) and comprised in a sector of said angular transition sector around the primary axis, the transition surface (26) has, at any given point (Pt(r)) of the transition section (126) from the primary transition point (128) to the secondary transition point (130), a slope (zt'(r)) of the local transition tangent which varies according to a linear function of the radial spacing (r) in an orthonormal coordinate system of the considered cutting plane.

7. The intraocular optical implant according to any one of the preceding claims, **characterized in that**, in any considered cutting plane containing the primary axis (01) and comprised in a sector of said angular transition sector around the primary axis, the transition section (126) is a section of conical curve.

8. The intraocular optical implant according to any one of the preceding claims, **characterized in that**, in the angular transition sector, the transition surface (26) extends over a surface of revolution having as axis of revolution the primary axis (01).

9. The intraocular optical implant according to any one of the preceding claims, **characterized in that** the angular transition sector extends over 360 degrees of angle around the primary axis (01).

10. The intraocular optical implant according to any one of the preceding claims, **characterized in that** one of the primary optical surface (18) and the secondary optical surface (24) extends radially outwardly around the other one with respect to the primary axis (O1).

11. The intraocular optical implant according to any one of the preceding claims, **characterized in that** either or both of the primary optical surface (18) and the secondary optical surface (20), is a surface of revolution around the primary axis (O1).

12. The intraocular optical implant according to any one of the preceding claims, **characterized in that** the transition surface (26) is preferably comprised between a primary transition circle and a secondary transition circle which have a difference in radius of 0.5 millimeter or less, preferably 0.15 millimeters or less, more preferably in the range from 0.05 to 0.15 millimeters.

13. The intraocular optical implant according to any one of the preceding claims, **characterized in that** the transition surface (26) is delimited by and comprised between a primary transition circle (28) common with the primary optical surface (18) and a secondary transition circle (30) common with the secondary optical surface (24).

14. The intraocular optical implant according to claim 13, **characterized in that** the primary transition circle (28) and the secondary transition circle (30) have a difference in radius of 0.5 millimeter or less, preferably 0.15 millimeter or less, more preferentially in the range from 0.05 to 0.15 millimeters.

15. The intraocular optical implant according to any one of the preceding claims, **characterized in that** the secondary base surface is a surface of revolution around the same primary axis (01) as the first primary base surface.

16. The intraocular optical implant according to any one of the preceding claims, **characterized in that** the first primary optical surface (18) and the secondary optical surface (24) are both convex surfaces, while the transition surface (26) is a concave surface.

FIG.1

FIG.2

FIG.3

FIG.4

FIG.5

FIG.6

FIG.7

FIG.8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2979663 A